(19) Europäisches Patentamt
European Patent Office
Office européen des brevets

(11) Publication number: 0 143 658
B1

(12) # EUROPEAN PATENT SPECIFICATION

(45) Date of publication of patent specification: 22.03.89

(51) Int. Cl.$^4$: C 07 D 501/22

(21) Application number: 84308266.0

(22) Date of filing: 28.11.84

(54) **Improvements in or relating to cephalosporin derivatives.**

(30) Priority: 01.12.83 US 556887

(43) Date of publication of application:
05.06.85 Bulletin 85/23

(45) Publication of the grant of the patent:
22.03.89 Bulletin 89/12

(84) Designated Contracting States:
GB NL SE

(58) References cited:
US-A-3 655 656

(73) Proprietor: ELI LILLY AND COMPANY
307, East McCarty Street
Indianapolis Indiana 46285 (US)

(72) Inventor: Engel, Gary Lowell
546 Leisure Lane Greenwood
Indiana 46142 (US)
Inventor: Indelicato, Joseph Michael
815 Winding Brook Lane
Greenwood Indiana 46142 (US)
Inventor: Rose, Harry Albert
1042 N. Graham Indianapolis
Indiana 46219 (US)
Inventor: McShane, Lawrence Joseph
418 David Lane Indianaplois
Indiana 46227 (US)
Inventor: Yang, Kuo Shang
340 Green Hill Court Greenwood
Indiana 46142 (US)

(74) Representative: Hudson, Christopher Mark et al
Erl Wood Manor
Windlesham Surrey GU20 6PH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 143 658 B1

Courier Press, Leamington Spa, England.

**Description**

This invention relates to a novel cephalosporin derivative, which is the crystalline hydrochloride salt of cephalexin monohydrate, and to its preparation from certain novel crystalline alkanol solvates.

Over the past decade, there has been much interest in the development of controlled release delivery systems involving the concept of an elementary osmotic pump, see, for instance, Theeuwes, F., "Elementary Osmotic Pump", *J. Pharm. Sci.,* Vol. 64, 1975, pp. 1987—1991, and U.S. Patent Specifications Nos. 3,845,770, 3,977,404, 4,008,719, 4,014,334, 4,016,880, 4,034,758, 4,036,227, 4,036,228, 4,096,238, 3,916,899, 4,111,203, 4,116,241, 4,160,020, 4,200,098 and 4,210,139.

In order to function in such a delivery system, the active agent must be sufficiently soluble in water and/or body fluids to permit development of sufficient differential osmotic pressure to effect release of the pharmaceutical from the device. The agent must also be of sufficient stability that it retains its pharmacological potency throughout the entire release period.

Although cephalexin monohydrate, i.e., 7 - (D - 2 - amino - 2 - phenylacetamido) - 3 - methyl - 3 - cephem - 4 - carboxylic acid monohydrate, see U.S. Patent Specification No. 3,655,656, has proven to be of immense value to mankind in the treatment of bacterial infections, its pharmacokinetic profile is such that it is most effective when administered using a multiple dosage regime. Thus, this compound would appear to be an ideal candidate for incorporation in an osmotic controlled release system of the type described above so that the frequency of dosing could be reduced.

Unfortunately, while cephalexin monohydrate is ideally suited for formulation into conventional dosage forms such as capsules and tablets, it does not lend itself to formulation as the active ingredient in dosage forms employing the osmotic pump technology, primarily because of its relatively low water solubility and the consequent low osmotic pressure of its solutions.

Attempts to solve this problem by forming the crystalline sodium salt of the monohydrate failed since, although the solubility of that material was acceptable (552 mg/ml in water), it rapidly degraded in solution, being stable for less than two hours at ambient temperature.

In addition, attempts using conventional procedures to prepare hydrochloride salts of cephalexin monohydrate in crystalline form failed totally. (In this context, it should not be forgotten that amorphous cephalosporin derivatives are generally unstable and that it is only the crystalline forms of cephalexin which are of sufficient stability to be of value in pharmaceutical formulations). Only by virtue of a chance observation as described below, and the development of a novel synthesis involving an unusual lattice transformation, did it finally become possible to synthesize the compound of the invention, i.e., crystalline cephalexin hydrochloride monohydrate.

According to the present invention there is provided crystalline cephalexin hydrochloride monohydrate.

This new crystalline salt is unusually soluble in water, forming a saturated aqueous solution containing 766 mg per ml of distilled water at 37°C. The pH of that solution is about 0.5. This solution exhibits an osmotic pressure of 143 atmospheres. This is to be contrasted with cephalexin monohydrate which has a solubility of only 12.6 mg per ml of distilled water and which solution exhibits a pH of 3.2 and an osmotic pressure of only 1.5 atmospheres.

Further, the high solubility of the hydrochloride monohydrate gives it the potential for providing high blood levels of cephalexin immediately upon administration in conventional pharmaceutical formulations such as tablets and capsules.

The novel crystalline salt of the invention has the following X-ray powder diffraction properties when measured with a 114.6 mm Debye-Scherrer camera using a nickel-filtered copper target tube of 1.5418 Å.

| Spacing, d(Å): | Relative intensities, $I/I_1$ |
|---|---|
| 14.03 | 1.00 |
| 7.08 | .33 |
| 5.42 | .33 |
| 4.63 | .73 |
| 4.41 | .27 |
| 4.31 | .13 |
| 4.17 | .47 |
| 3.99 | .13 |
| 3.78 | .40 |
| 3.70 | .27 |
| 3.55 | .53 |
| 3.38 | .20 |
| 3.21 | .07 |
| 3.12 | .07 |
| 3.03 | .07 |
| 2.85 | .13 |

| Spacing, d(Å): | Relative intensities, $I/I_1$ |
|---|---|
| 2.73 | .03 |
| 2.65 | .03 |
| 2.59 | .03 |
| 2.53 | .13 |
| 2.37 | .20 |
| 2.29 | .13 |
| 2.18 | .03 |
| 2.14 | .03 |
| 1.996 | .13 |
| 1.959 | .07 |

As intimated previously, it was surprisingly discovered that the crystalline cephalexin hydrochloride monohydrate could be derived from the corresponding crystalline $C_{1-4}$ alkanol hydrochloride solvates.

The Applicants had prepared the novel crystalline ethanol solvate of cephalexin hydrochloride and were surprised by its instability. They discovered that, under certain conditions of relative humidity, a most unusual transformation was taking place. Thus, under those conditions, water molecules were displacing the ethanol molecules from the crystal lattice, thus providing the monohydrate of the invention. Interestingly, the X-ray powder diffraction patterns of the two solvates, although similar, were not identical, so that after the displacement has occurred there is a molecular rearrangement of the lattice framework.

Further study showed that this same displacement occurred to a greater or lesser extent with all of the crystalline $C_{1-4}$ alkanol solvates.

According to a second aspect of the invention there is provided a process for preparing crystalline cephalexin hydrochloride monohydrate which comprises hydrating a crystalline cephalexin hydrochloride $C_{1-4}$ alkanol solvate by exposing the solvate to an atmosphere, typically air, having a relative humidity of from about 10 to about 50%. Temperatures of from about 10 to about 50°C provide satisfactory conversion rates. In general, the higher the temperature, the lower should be the relative humidity.

Hydration of the alkanol solvate is most facile with the methanol and ethanol solvates. Indeed, hydration of the propanol and butanol solvates is much more difficult to drive to the completion.

The crystalline alkanol solvates of the invention can be prepared by adding an excess of hydrogen chloride or hydrochloric acid to an alkanolic suspension of cephalexin monohydrate. When preparing the $C_{1-2}$ alkanol solvates, it is preferred to use anhydrous, i.e., gaseous hydrogen chloride during the formation of the alkanol solvate. On the other hand, the $C_{3-4}$ alkanol solvates are preferably formed using aqueous hydrochloric acid.

The cephalexin hydrochloride alkanol solvate typically crystallizes out of solution. The crystallization can be assisted by chilling, preferred crystallization temperatures being from 15 to 25°C, most preferably from 20 to 22°C, or by addition of an anti-solvent such as a hydrocarbon solvent, for example hexane. Seeding with crystals of the hydrochloride monohydrate or alkanolate may also assist the crystallization process.

The crystalline cephalexin hydrochloride alkanol solvates are new compositions of matter, and are provided in one aspect of the invention.

While the crystalline cephalexin hydrochloride alkanol solvate intermediates of the invention are relatively stable under anhydrous conditions, if they are exposed to an atmosphere having a relative humidity greater than about 10% the solvate becomes unstable and converts to the monohydrate polymorph of the invention. The rate of conversion varies depending upon the particle size of the solvate, the relative humidity to which it is exposed, and the ambient temperature. If the solvate intermediate is subjected to an atmosphere having a high relative humidity, the material does not form the monohydrate crystalline structure provided by this invention, but instead becomes an amorphous mass.

In a preferred embodiment, the ethanol hydrochloride solvate is exposed to air having a relative humidity of from 20 to 45%, at a temperature of from 20 to 50°C. Under such conditions conversion to the hydrochloride monohydrate crystalline form of this invention is substantially complete after one to fifteen days.

The cephalexin hydrochloride monohydrate crystalline form of the invention is useful as an orally active antibacterial agent, and is particularly well suited for formulation in sustained release formulations as described previously or in conventional dosage forms such as tablets or capsules. Thus, the compound can be admixed with conventional carriers and excipients such as sucrose, polyvinylpyrrolidone, stearic acid, starch, and the like and encapsulated or, if desired, the formulation can be compressed into tablets. A preferred embodiment is a tablet adapted for human chemotherapy which provides for substantially immediate release of the active ingredient into the biological system.

Such pharmaceutical formulations will contain from 10 to 98% by weight of active ingredient, for example from 200 to 1200 mg of active ingredient, and will be administered to a human subject at the rate of one or more doses each day for the control and prevention of bacterial infections. The compound can

additionally be admixed with polymers made from polymerizable materials such as methacrylate esters, glycols, hydroxy acids such as lactic acid and the like, and molded into tablets or the like.

While the cephalexin hydrochloride monohydrate crystalline form can be formulated for oral administration employing conventional encapsulation and tableting technology, as described above, it is ideally suited to formulation as a controlled release dosage form, especially employing osmotically actuated technology for rate-controlled drug delivery. For a compound to be suitable for delivery via an osmotic pump, it must be sufficiently soluble in water or similar fluid to be solubilized over a period of time sufficiently long to provide continuous delivery over a desired period at pharmacologically effective rates, and sufficiently stable when in solution to remain therapeutically efficacious over the entire period of administration. The compound of this invention uniquely satisfies these requirements of solubility, osmotic pressure and stability.

The amount of cephalexin hydrochloride monohydrate present in the osmotically-driven delivery device is not critical but typically is an amount equal to or greater than the amount necessary to osmotically operate the device for the desired period of drug release so that the desired therapeutic level of active agent is achieved for the desired period of time.

Moreover, the cephalexin hydrochloride monohydrate crystalline structure of this invention can easily be produced in a pharmaceutically acceptable state of purity in that the level of $C_{1-2}$ alkanol contaminant can be reduced below two, normally below one, percent by weight. The novel monohydrate polymorph of this invention therefore normally exists in a pharmaceutically-acceptable state of purity greater than 98 percent, preferably and typically greater than 99 percent by weight.

According to a further aspect of the invention, there is provided a pharmaceutical formulation which comprises as the active ingredient crystalline cephalexin hydrochloride monohydrate associated with one or more pharmaceutically-acceptable carriers or excipients therefor.

The amount of cephalexin hydrochloride monohydrate which is antibacterially effective is from 1 to 30 mg/kg of animal body weight. While cephalexin hydrochloride monohydrate will display an activity profile very similar to that of cephalexin monohydrate, it is likely that higher blood levels and a more rapid onset of action will be enjoyed with cephalexin hydrochloride monohydrate than with the current commercial cephalexin monohydrate, due to its unusually greater solubility. Thus, the compound of the invention has great potential as an immediate release tablet composition.

The invention will now be further illustrated with reference to the following examples.

Example 1

Cephalexin hydrochloride ethanol solvate

Cephalexin monohydrate (100 g) was suspended in 300 ml of absolute ethanol. The suspension was stirred at 25°C while hydrogen chloride was bubbled through the suspension until all particles were in solution. The reaction mixture was stirred at 25°C for two hours, and then cooled to 0°C and stirred for an additional two hours. The crystalline product was collected by filtration and washed with 200 ml of 1:1 (v/v) ethanol-ethyl acetate and then with 200 ml of ethyl acetate. The product was identified as cephalexin hydrochloride ethanol solvate. Yield 53 grams.

NMR: ($D_2O$): δ1.02 (t, 3H);
δ2.02 (s, 1H);
δ3.23 (q, 2H);
δ3.65 (q, 2H);
δ5.0 (d, 1H);
δ5.3 (s, 1H);
δ5.61 (d, 1H);
δ7.59 (s, 5H).

X-ray powder diffraction carried out with a diffractometer having a nickel-filtered copper target tube of 1.5405 Å.

| Spacing, d(Å) | Relative intensities, $I/I_1$ |
|---|---|
| 14.48 | 1.00 |
| 10.04 | .005 |
| 9.16 | .01 |
| 8.58 | .02 |
| 7.34 | .095 |
| 6.10 | .055 |
| 5.75 | .05 |
| 5.48 | .175 |
| 5.08 | .01 |
| 4.62 | .035 |
| 4.32 | .035 |
| 4.02 | .025 |
| 3.97 | .025 |
| 3.78 | .01 |
| 3.72 | .035 |
| 3.68 | .06 |
| 3.43 | .01 |
| 3.36 | .06 |
| 3.16 | .035 |
| 3.04 | .035 |
| 2.74 | .01 |
| 2.54 | .01 |
| 2.52 | .025 |
| 2.45 | .01 |
| 2.42 | .015 |

Example 2

Cephalexin hydrochloride monohydrate

To a stirred suspension of 45 kg of cephalexin monohydrate in 168 liters of absolute ethanol were added portion-wise over thirty minutes 5.7 kg of hydrogen chloride. The reaction mixture was stirred at 25°C for thirty minutes, and then was cooled to 10°C and stirred for an additional two hours. The crystalline precipitate that had formed was collected by filtration and washed with 24 liters of 1:1 (v/v) ethanol-hexane, and finally with 22 liters of hexane. The filter cake was shown by NMR to be cephalexin hydrochloride ethanol solvate (NMR consistent with that reported in Example 1).

Elemental Analysis calculated for ethanol solvate:

$$C_{16}H_{17}N_3O_4S \cdot HCl \cdot CH_3CH_2OH$$

| | |
|---|---|
| Theory: | C, 50.29; H, 5.63; N, 9.77; S, 7.46; Cl, 8.25; |
| Found: | C, 50.03; H, 5.45; N, 9.84; S, 7.35; Cl, 8.37. |

The ethanol solvate filter cake from above was exposed for two weeks to an atmosphere of air of about 35% relative humidity at a temperature of about 25—30°C to provide 31.76 kg of cephalexin hydrochloride monohydrate.

| | |
|---|---|
| NMR ($D_2O$): | δ2.06 (s, 3H) |
| | δ3.30 (q, 2H) |
| | δ5.0 (d, 1H) |
| | δ5.32 (s, 1H) |
| | δ5.68 (d, 1H) |
| | δ7.61 (s, 5H). |
| IR (KBr): | 3290 $cm^{-1}$ |
| | 3120 |
| | 1760 |
| | 1710 |
| | 1680 |
| | 1560 |
| | 1490 |

Karl Fisher water analysis: 4.48% (n=4), consistent with the presence of approximately one mole of water. Residual ethanol determined to be 0.68%.

Elemental Analysis Calculated for cephalexin hydrochloride monohydrate:

$C_{16}H_{17}N_3O_4S \cdot HCl \cdot H_2O$

Theory: C, 47.82; H, 5.02; N, 10.46; S, 7.98; Cl, 8.82.
Found: C, 48.03; H, 4.82; N, 10.27; S, 7.87; Cl, 8.90.

Differential thermal analysis demonstrated the compound has a large broad endotherm at 109°C which appears to indicate a loss of volatile materials, and a sharp exotherm at 202°C which appears to indicate decomposition of the compound. A thermal gravimetric analysis showed a weight loss beginning at 63°C which resulted in a 5.7% weight loss at 135°C. At 150°C another weight loss began and continued through decomposition. The compund demonstrated an X-ray powder diffraction pattern consistent with that reported above for cephalexin hydrochloride monohydrate.

Example 3
The effect of humidity on the rate of change of cephalexin hydrochloride ethanol solvate to cephalexin hydrochloride monohydrate was studied by X-ray diffraction of samples of the ethanol solvate after storage at 25°C in chambers of different relative humidities. The change from ethanol solvate to monohydrate was followed by observing the disappearance of an X-ray minimum having a d value of about 7.34 Å. The results of the study are presented in Table I.

TABLE I
Disappearance of 7.34 Å X-ray maximum with time at various humidities at 25°C

| | Relative humidity (%) | | | |
|---|---|---|---|---|
| Time | 0 | 20 | 32 | 44 |
| 0 hours | 19 units | 19 units | 19 units | 19 units |
| 24 | 18 | 10 | 7 | 2 |
| 48 | 17 | 8 | 5 | 1 |
| 72 | — | 6 | 4 | — |
| 144 | — | 4 | 2 | 0 |
| 260 | — | 2 | 1 | — |
| 888 | — | 1 | 0 | — |

Note: "—" means that no reading was taken.

A sample of the ethanol solvate held at 70% relative humidity was totally dissolved within twenty-four hours.

Example 4
Stability of cephalexin hydrochloride monohydrate
A sample of cephalexin hydrochloride monohydrate from Example 2 was analyzed by high pressure liquid chromatography and shown to contain 84.6% cephalexin. (This is equivalent to a purity of about 99.2% for the cephalexin hydrochloride monohydrate, the remainder being substantially all ethanol). Samples of this material were stored at various temperatures for a prolonged period of time. The samples were assayed periodically by high pressure liquid chromatography (HPLC) and by Karl Fischer (KF) titration. The results of the study are given in the following Table II:

TABLE II
Stability of bulk cephalexin hydrochloride monohydrate

| Time of assay | 5°C* | | | 25°C | | | 40°C | | | 50°C | | |
|---|---|---|---|---|---|---|---|---|---|---|---|---|
| | HPLC+ | KF | Total | HPLC+ | KF | Total | HPLC+ | KF | Total | HPLC+ | KF | Total |
| initial | 84.01 | 5.58 | 89.59 | 84.01 | 5.58 | 89.59 | 84.01 | 5.58 | 89.59 | 84.01 | 5.58 | 89.59 |
| 1.5 mo. | 81.42 | 6.28 | 87.70 | 84.01 | 5.66 | 89.67 | 84.46 | 5.47 | 89.93 | 85.06 | 5.50 | 90.56 |
| 3 mo. | 80.34 | 5.25 | 85.59 | 84.42 | 4.81 | 89.23 | 83.74 | 4.70 | 88.44 | 84.41 | 4.63 | 89.04 |
| 6 mo. | 78.86 | 5.60 | 87.40 | 86.52 | 4.23 | 90.75 | 86.45 | 4.14 | 90.59 | 86.34 | 4.38 | 90.72 |
| 9 mo. | 80.95 | 6.20 | 87.15 | 84.10 | 5.28 | 84.38 | 83.82 | 5.11 | 88.93 | 83.16 | 5.19 | 88.35 |
| 12 mo. | 77.77 | 9.07 | 86.84 | 83.33 | 6.97 | 90.30 | 84.66 | 4.81 | 89.47 | 83.83 | 4.71 | 88.54 |

*This sample was placed in a plastic container permeable to moisture and stored in a refrigerator in which the atmosphere was quite moist. It is believed that this constant exposure to high humidity contributed to the observed decomposition and increase in water content.

+reported in terms of anhydrous cephalexin.

Example 5

Cephalexin hydrochloride methanol solvate

Anhydrous methanol (100 ml) was cooled to 5°C and treated with gaseous hydrogen chloride (8 g). Cephalexin monohydrate (35.2 g) was then added at room temperature. Solution of the cephalexin monohydrate occurred followed (within 15 minutes) by formation of a thick slurry of the title compound. Yield 18.0 g.

The X-ray diffraction pattern of this material, carried out in the presence of mother liquor, with a 114.6 mm Debye-Scherrer camera using a nickel-filtered copper target tube of 1.5418 Å, in a sealed glass capillary tube is given below.

| Spacing, d(Å) | Relative intensities, $I/I_1$ |
|---|---|
| 13.97 | 1.00 |
| 7.10 | .49 |
| 6.50 | .01 |
| 6.08 | .01 |
| 5.65 | .15 |
| 5.40 | .40 |
| 4.69 | .43 |
| 4.51 | .03 |
| 4.38 | .03 |
| 4.23 | .50 |
| 4.04 | .03 |
| 3.78 | .66 |
| 3.61 | .44 |
| 3.48 | .29 |
| 3.41 | .18 |
| 3.27 | .04 |
| 3.07 | .12 |
| 2.93 | .06 |
| 2.86 | .18 |
| 2.74 | .03 |
| 2.61 | .06 |
| 2.56 | .13 |
| 2.42 | .10 b |
| 2.31 | .13 |
| 2.22 | .06 |
| 2.15 | .04 |
| 2.05 | .07 |
| 1.992 | .01 |
| 1.946 | .03 |
| 1.885 | .06 b |
| 1.790 | .01 |
| 1.748 | .03 |
| 1.708 | .01 |

Example 6

Cephalexin hydrochloride monohydrate

Hydrogen chloride gas (7.0 g) was dissolved in methanol (100 ml) at room temperature. The methanol was anhydrous (Karl Fischer analysis showed less than 0.12% by weight of water). Cephalexin monohydrate (35.2 g) in solid form was then added to the reaction mixture. Dissolution occurred. Crystallization of cephalexin hydrochloride methanol solvate occurred approximately fifteen minutes after seeding with cephalexin hydrochloride monohydrate. Heat of crystallization caused the temperature to rise from 22 to 30°C. After 3 hours at room temperature, the product was filtered off, and then washed with cold methanol. NMR studies showed the initial formation of the methanol solvate.

The methanol solvate prepared above was exposed for three days to an air atmosphere having a relative humidity of about 35% and at a temperature of 28°C to provide 18.1 grams of crystalline cephalexin hydrochloride monohydrate having an NMR and IR identical with the product of Example 2.

Example 7

Cephalexin hydrochloride isopropanolate

Cephalexin dimethylformamide disolvate (300 g) was slurried in isopropanol (2.215 L) and cooled to 13°C. Concentrated hydrochloric acid (190 ml) was added rapidly, dropwise, to the reaction mixture at a temperature between 13 and 17°C. After addition was complete, a yellow solution was formed. That solution was warmed to 20°C and slowly stirred. Cephalexin hydrochloride isopropanol solvate crystallized out in the form of a thick slurry which was stirred at room temperature for 2 hours, treated with hexane, 200 ml of isopropanol added and then stirred for a further 3 hours at room temperature, cooled and then filtered. The product was then washed with 1:1 (by volume) isopropanol/hexane (2×100 ml). Yield 254.1 g.

The X-ray diffraction pattern obtained for this material, measurement obtained with a Debye-Scherrer camera using a nickel-filtered copper target tube of 1.5418 Å is given below:

| d(Å) | Relative intensities, $I/I_1$ |
|---|---|
| 14.61 | 1.00 |
| 7.44 | .17 |
| 6.15 | .06 |
| 5.70 | .27 b |
| 4.68 | .30 |
| 4.40 | .12 |
| 4.29 | .12 |
| 4.10 | .09 |
| 3.97 | .06 |
| 3.83 | .33 |
| 3.69 | .12 |
| 3.53 | .09 |
| 3.43 | .18 |
| 3.25 | .06 |
| 3.04 | .09 |
| 2.96 | .02 |
| 2.80 | .08 |
| 2.69 | .03 |
| 2.56 | .09 |
| 2.50 | .02 |
| 2.43 | .02 |
| 2.35 | .02 |
| 2.27 | .08 |
| 2.21 | .03 |
| 2.13 | .02 |
| 2.05 | .03 |

Example 8

Cephalexin hydrochloride monohydrate

The isopropanolate product (35 g) of Example 7 was loaded into the fluid bed drier sold under the trade name "Lab-Line" and allowed to humidify at temperatures ranging between 24 and 27°C. After 18.5 hours NMR studies of the final product showed that some 54% of the starting isopropanolate had been converted to the crystalline hydrochloride monohydrate.

Example 9

Cephalexin hydrochloride *n*-propanol solvate

Cephalexin monohydrate (35.2 g) was slurried in anhydrous *n*-propanol (150 ml) cooled to approximately 10°C and treated with gaseous hydrogen chloride (6.1 g). The solution thus formed was seeded with the isopropanolate solvate formed in Example 7. Further *n*-propanol 50 ml) was added and the reaction mixture stirred at room temperature for a further 3 hours, whereupon the desired *n*-propanol solvate crystallized out as a slurry. The slurry was then cooled for 2 hours and the title compound isolated, yield 39.1 g. NMR showed the material to be the *n*-propanolate solvate. The X-ray diffraction pattern of this material, measurement carried out as with methanolate, for the *n*-propanolate is given below:

| d(Å) | I/I$_1$ |
|---|---|
| 14.92 | 1.00 |
| 7.58 | .41 |
| 6.27 | .06 |
| 5.96 | .13 |
| 5.57 | .34 |
| 4.65 | .59 |
| 4.38 | .41 |
| 4.23 | .44 |
| 4.06 | .41 |
| 3.78 | .59 |
| 3.66 | .06 |
| 3.50 | .13 |
| 3.46 | .22 |
| 3.41 | .16 |
| 3.23 | .06 |
| 3.08 | .11 |
| 2.96 | .03 |
| 2.81 | .03 |
| 2.76 | .05 |
| 2.67 | .02 |
| 2.60 | .06 |
| 2.53 | .06 |
| 2.47 | .03 |
| 2.37 | .03 |
| 2.33 | .03 |
| 2.25 | .02 |
| 2.18 | .11 |
| 2.03 | .02 |
| 1.986 | .03 |
| 1.904 | .05 |
| 1.882 | .03 |
| 1.820 | .02 |
| 1.777 | .02 |
| 1.735 | .02 |

Example 10
Cephalexin hydrochloride monohydrate

The product of Example 9 was allowed to dry in air at 30 to 35°C under a relative humidity of about 35%. After 15 days, NMR studies showed that approximately 73% of the *n*-propanolate solvate had transformed to the monohydrate title compound

Example 11
Tablet for immediate release product

| Ingredient | Amount |
|---|---|
| Cephalexin hydrochloride monohydrate of Example 2 (850 mcg cephalexin/mg) | 617.7 mg |
| 1-Ethenyl-2-pyrrolidone homopolymer (=Povidone) | 12.6 mg |
| Carboxymethylcellulose sodium (cross linked) | 26.0 mg |
| Stearic acid | 12.6 mg |
| Magnesium stearate | 6.3 mg |

The cephalexin hydrochloride monohydrate was granulated with povidone (=1 - ethenyl - 2 - pyrrolidone homopolymer) in dichloromethane. After drying and sizing, the granules were blended to uniformity with the remaining ingredients and compressed.

Example 12
Tablet formulation

| Ingredient | Amount |
|---|---|
| Cephalexin hydrochloride monohydrate (Example 2) | 617.7 mg |
| Povidone | 12.6 mg |
| Emcosoy® (excipient derived from defatted soybeans; Edward Mendell Co., Inc.) | 26.0 mg |
| Stearic acid | 12.6 mg |
| Magnesium stearate | 6.3 mg |

The above ingredients were blended as described in Example 11 and compressed into tablets.

Example 13

| Ingredient | Amount |
|---|---|
| Cephalexin hydrochloride monohydrate (Example 2) | 617.7 mg |
| Povidone | 12.6 mg |
| Starch | 26.0 mg |
| Stearic acid | 12.6 mg |
| Magnesium stearate | 6.3 mg |

The ingredients were blended by the method described in Example 11. The resulting tablets were coated with hydroxypropyl methyl cellulose for use as immediate release antibacterial pharmaceutical form.

Example 14
Capsule formulation

| Ingredient | Amount |
|---|---|
| Cephalexin hydrochloride monohydrate | 450 mg |
| Povidone | 10 mg |
| Magnesium stearate | 5 mg |

The ingredients were blended to uniformity and placed into an elongated gelatin capsule.

**Claims for the Contracting State: GB**

1. Crystalline cephalexin hydrochloride monohydrate.
2. A pharmaceutical formulation comprising as the active ingredient crystalline cephalexin hydrochloride monohydrate associated with one or more pharmaceutically-acceptable carriers or excipients therefor.
3. Crystalline cephalexin hydrochloride monohydrate for use as a pharmaceutical.
4. A process for preparing crystalline cephalexin hydrochloride monohydrate, which process comprises hydrating a crystalline hydrochloride $C_{1-4}$ alkanol solvate by exposing the solvate to an atmosphere having a relative humidity of from 10 to 50%.
5. A process according to claim 4, in which a crystalline hydrochloride $C_{1-2}$ alkanol solvate is hydrated.
6. A process according to claim 5, wherein the solvate is the ethanol solvate.
7. A crystalline cephalexin hydrochloride $C_{1-4}$ alkanol solvate.
8. Crystalline cephalexin hydrochloride ethanol solvate.

**Claims for the Contracting States: NL, SE**

1. Crystalline cephalexin hydrochloride monohydrate.
2. A pharmaceutical formulation comprising as the active ingredient crystalline cephalexin hydrochloride monohydrate associated with one or more pharmaceutically-acceptable carriers or excipients therefor.
3. Crystalline cephalexin hydrochloride monohydrate for use as a pharmaceutical.

**Patentansprüche für den Vertragsstaat: GB**

1. Kristallines Cephalexinhydrochloridmonohydrat.
2. Pharmazeutische Formulierung, dadurch gekennzeichnet, daß sie kristallines Cephalexinhydrochloridmonohydrat als Wirkstoff in Verbindung mit einem oder mehreren pharmazeutisch annehmbaren Trägern oder Hilfsstoffen hierfür enthält.

3. Kristallines Cephalexinhydrochloridmonohydrat zur Verwendung als Pharmzeutikum.

4. Verfahren zur Herstellung von kristallinem Cephalexinhydrochloridmonohydrat, dadurch gekennzeichnet, daß ein kristallines Cephalexinhydrochlorid - $C_1$—$C_4$ - alkanolsolvat durch Behandlung in einer Atmosphäre mit einer relativen Feuchtigkeit von 10 bis 50% einer Hydratation unterzogen wird.

5. Verfahren nach Anspruch 4, dadurch gekennzeichnet, daß ein kristallines Cephalexinhydrochlorid - $C_1$—$C_2$ - alkanolsolvat einer Hydratation unterzogen wird.

6. Verfahren nach Anspruch 5, dadurch gekennzeichnet, daß das Solvat das Ethanolsolvat ist.

7. Kristallines Cephalexinhydrochlorid - $C_1$—$C_4$ - alkanolsolvat.

8. Kristallines Cephalexinhydrochloridethanolsolvat.

**Patentansprüche für die Vertragsstaaten: NL, SE**

1. Kristallines Cephalexinhydrochloridmonohydrat.

2. Pharmazeutische Formulierung, dadurch gekennzeichnet, daß sie kristallines Cephalexinhydrochloridmonohydrat als Wirkstoff in Verbindung mit einem oder mehreren pharmazeutisch annehmbaren Trägern oder Hilfsstoffen hierfür enthält.

3. Kristallines Cephalexinhydrochloridmonohydrat zur Verwendung als Pharmazeutikum.

**Revendications pour l'Etat Contractant: GB**

1. Chlorhydrate de céphalexine cristallin à une molécule d'eau.

2. Formulation pharmaceutique comprenant, comme ingrédient actif, du chlorhydrate de céphalexine cristallin à une molécule d'eau en association avec un ou plusieurs excipients ou supports pharmaceutiquement acceptables pour cette formulation.

3. Chlorhydrate de céphalexine cristallin à une molécule d'eau destiné à être utilisé comme produit pharmaceutique.

4. Procédé de préparation de chlorhydrate de céphalexine cristallin à une molécule d'eau, caractérisé en ce qu'il consiste à hydrater un produit de solvatation d'alcanol en $C_1$—$C_4$ d'un chlorhydrate cristallin en exposant ce produit de solvatation à une atmosphère ayant une teneur en humidité relative de 10 à 50%.

5. Procédé selon la revendication 4, caractérisé en ce que le produit de solvatation d'un alcanol en $C_1$—$C_2$ d'un chlorhydrate cristallin est hydraté.

6. Procédé selon la revendication 5, caractérisé en ce que le produit de solvatation est le produit de solvatation de l'éthanol.

7. Produit de solvatation d'un alcanol en $C_1$—$C_4$ d'un chlorhydrate de céphalexine cristallin.

8. Produit de solvatation d'éthanol d'un chlorhydrate de céphalexine cristallin.

**Revendications pour les Etats Contractants: NL, SE**

1. Chlorhydrate de céphalexine cristallin à une molécule d'eau.

2. Formulation pharmaceutique comprenant, comme ingrédient actif, du chlorhydrate de céphalexine cristallin à une molécule d'eau en association avec un ou plusieurs excipients ou supports pharmaceutiquement acceptables pour cette formulation.

3. Chlorhydrate de céphalexine cristallin à une molécule d'eau destiné à être utilisé comme produit pharmaceutique.